# EUROPEAN PATENT APPLICATION

(11) **EP 1 582 217 A1**
(43) Date of publication of application: **05.10.2005**
(21) Application number: 03773443.1
(22) Date of filing: 14.11.2003
(51) Int. Cl.: A61K 39/02, A61K 47/48

(54) **METHOD OF OBTAINING CONJUGATE VACCINES AND VACCINE COMPOSITIONS CONTAINING SAME**

(30) Priority: 14.11.2002 CU 2572002
(71) Applicant: Instituto Finlay, Centro de Investigacion-Produccion de vacunas y sueros, Ciudad Habana 11600 (CU)
(72) Inventor: CABRERA BLANCO, Osmir, 12100 Ciudad de la Habana (CU); CUELLO PEREZ, Maribel, 12100 Ciudad de la Habana (CU); SIERRA GONZALEZ, Victoriano Gustavo, 16017 Ciudad de la Habana (CU); SOTO RODRIGUEZ, Carmen Rosa, 12100 Ciudad de la Habana (CU); MARTINEZ POZO, Miguel Ernesto, 10800 Ciudad de la Habana (CU)
(74) Representative: Winckels, Johannes Hubertus F.
(86) International application number: PCT/CU2003/000013
(87) International publication number: WO 2004/043489

(57) **Abstract**

This invention is related to biotechnology, specially with the methods of chemical conjugation of antigenic molecules. Hereby enclosed there is a description of a method for the conjugation of saccharides antigens. The invention is based on the generation of amino groups in a saccharide structure, from which one saccharide with two different functional groups is obtained. This method could be used to fix proteins to saccharides. Also included are the conjugates which happen to be effective against bacterial diseases, which were also obtained through the aforementioned method.

## Description

This invention is related to biotechnology, specially with the methods of chemical conjugation of antigenic molecules. Animal studies had shown that bacterial polysaccharides were the most virulent antigens. Consequently, several purified capsular polysaccharide vaccines were created, but various clinical trials proved that these were thymus-independent molecules (TI), which means that they are not immunogenic in children below two years of age (Lepow, M.L., et al. Persistence of antibody following immunization of children with group A and group C meningococcal polysaccharides. Pediatrics. 1977.60:673-80).

In order to obtain vaccines that are more effective against different bacteria in younger children, several working strategies were developed, among them the covalent bonding between polysaccharides or its fragments and carrier proteins. So is the case for the already existing commercial vaccines against Haemophilus influenzae Type B (Hib). (Decker, M.D., et al. Comparative trial in infants for four conjugates Haemophilus Influenzae Type B vaccines. J. Pediatrics. 1992. 120 (2) part I: 184-189.; Eskola, J. et al.. Ten year's experience with Haemophilus influenzae Type B (Hib) conjugate vaccines in Finland. Rev. Med. Microbiol. 1996. 7:231-41).

Conjugation works have also been developed in other molecules, such as the Neisseria meningitidis B or C polysaccharide (Richmond, P.C. et al. Meningococcal serogroup C conjugate vaccine is immunogenic in infancy and primes for memory. J. Infect. Dis. 1999. 179:1569-1572; Borrow, R. Et al. Meningococcal serogroup C-specific IgG antibody responses and serum bactericidal titres in children following vaccination with a meningococcal A/C polysaccharide vaccine. FEMS Immunology and Medical Microbiology. 2000. 28: 79-85).

Current literature includes many articles about polysaccharide antigens conjugated with proteins through different methods, as, for example, Patent USA No. 4 673 574, which describes the use of a bacterial capsular polysaccharide conjugated with a carrier protein to form an immunogenic compound. Another example is described in Patent USA No. 4 356 170, using a polysaccharide obtained from a microbial culture of N. Meningitidis serogroup A that merges with an immunogenic protein by means of a covalent bond.

In general, all current methods used for the conjugation of polysaccharides work in two fundamental directions. The first is related to the obtention of polysaccharides fragments; and the second to the areas where the functional groups that exist in the polysaccharide chain are used (Martinez JC. Et al. Preparación de Oligosacáridos de N. Meningitidis serogrupo C por hidrólisis ácida. Biotecnologia aplicada. 1999. 16:25-28; Pawlowski A. Et al. Preparation of pneumococcal capsular polysaccharide-protein conjugate vaccines utilizing new fragmentation and conjugation technologies. Vaccine. 2000. 18(8): 1873-85; Cabrera O. and col. Fragmentación del polisacárido de Neisseria meningitidis serogrupo C para su uso en vacunas conjugadas. VacciMonitor. 2001.10 (3): 1-6).
The chart A expressed in a summarized way the main characteristics of the used methods until the present time.

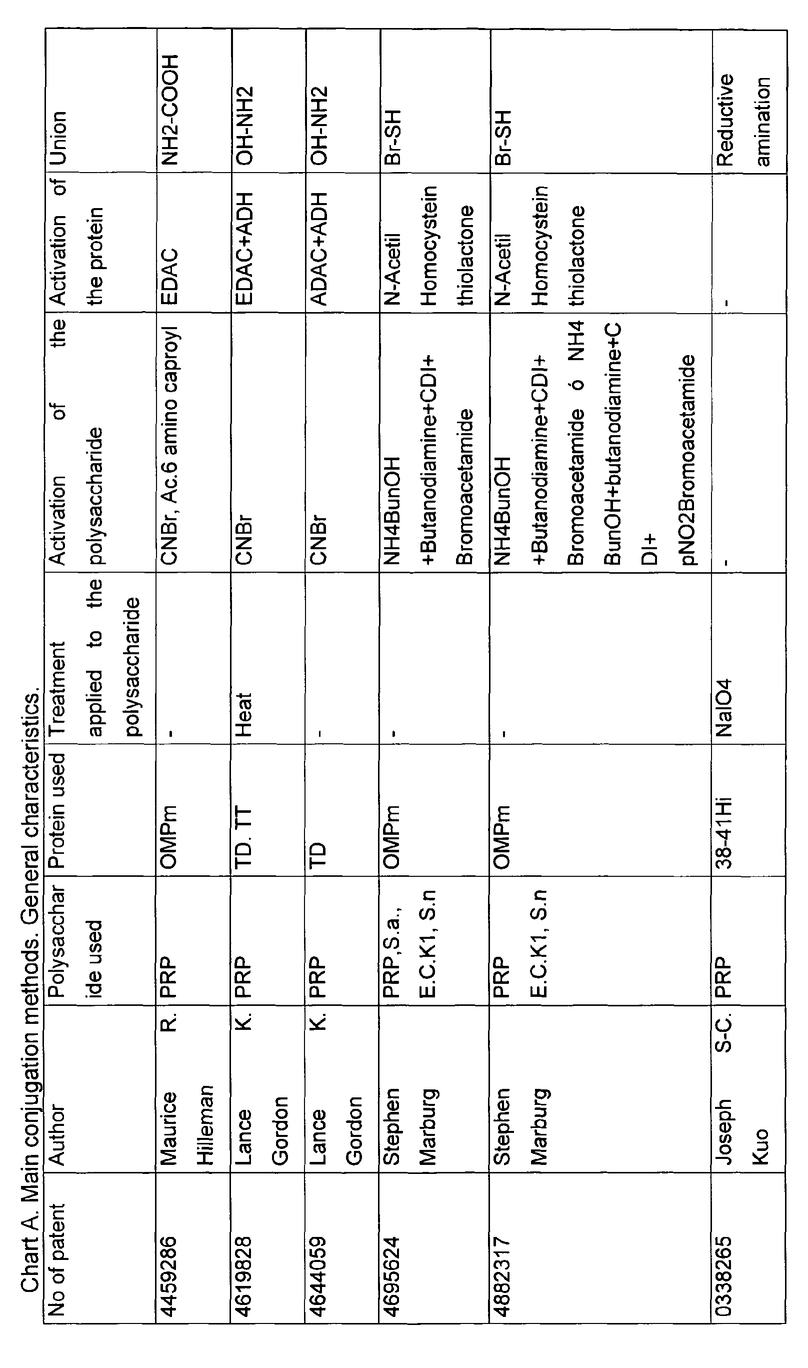

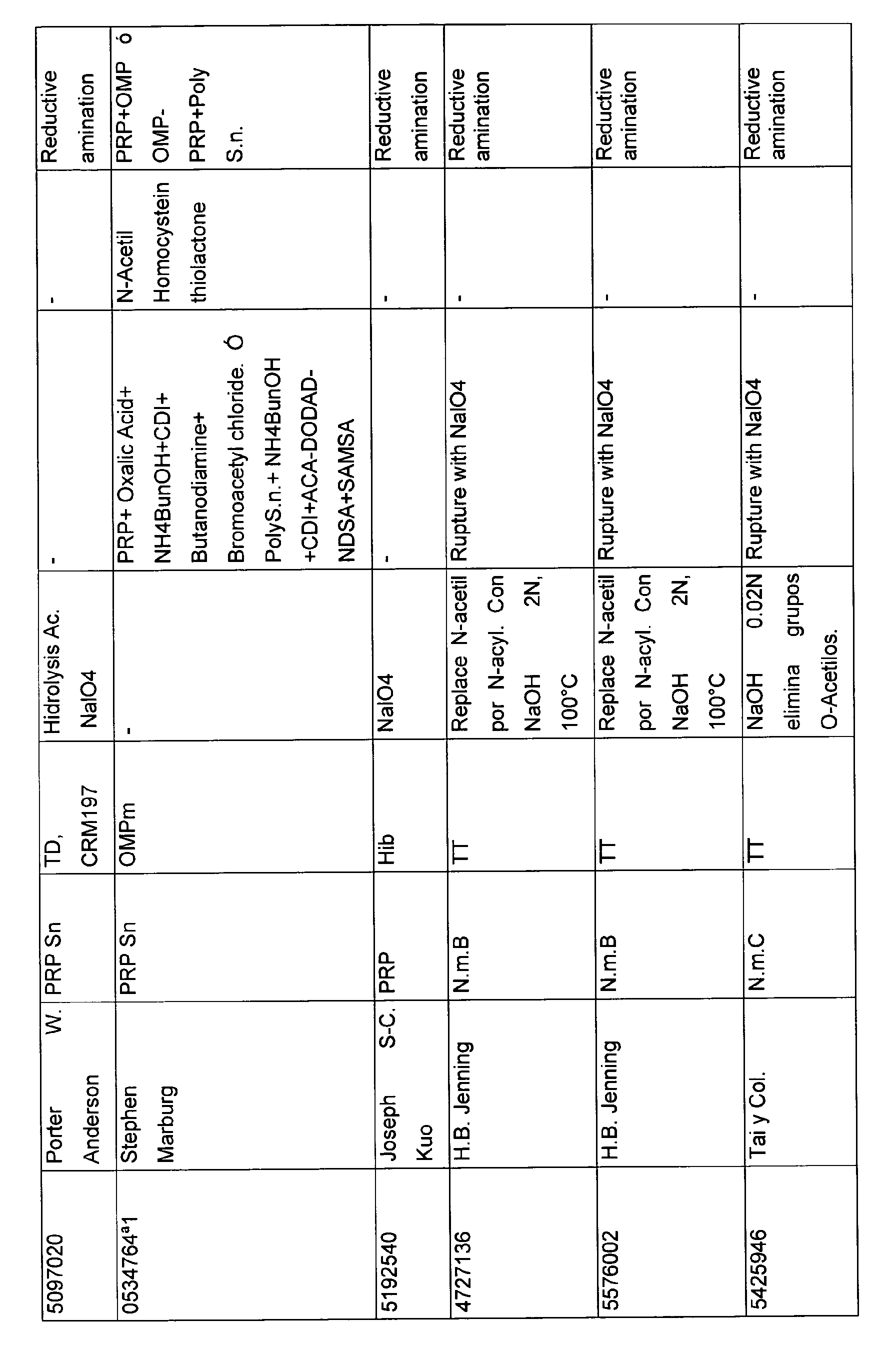

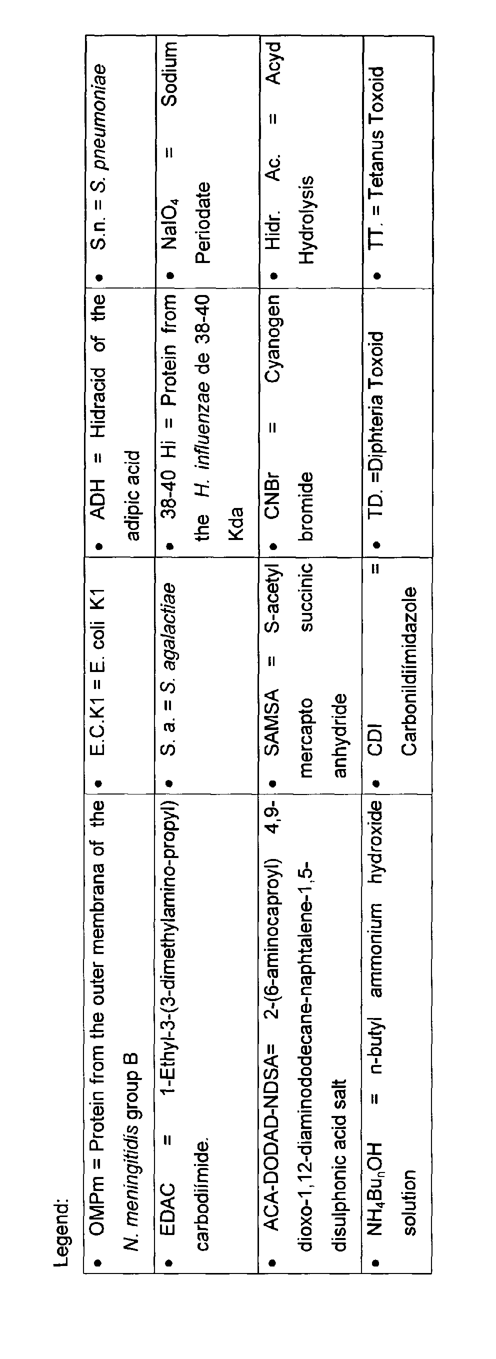

There are several methods to conjugate polysaccharides with carrier proteins. In case the polysaccharide is the PRP of H. Influenzae, we have found out a method whereby the tetrabutylammonium of the PRP is formed first. Afterwards the hydroxyls groups are activated with carbonyl diimidazole (CDI); the 1.4 di-amino butane is introduced, and finally we introduce a bromide, which will be the one responsible for the fusion with the protein. The protein to be used for the conjugation is previously activated with N-acetylhomocysteine thio-lactone, which leads to the creation of an SH group that reacts to the bromide of the polysaccharide.

From all conjugation methods using N. Meningitidis polysaccharides, we have found some where polysaccharides undergo a structural modification through a basic hydrolysis using NaOH in order to eliminate the O-Acetyl groups that exist in them. We already proved that the concentration of NaOH 0.02 N can eliminate these groups from the meningococcus "C" polysaccharides. Other studies have been carried out using meningococcus "B" polysaccharides with NaOH 2M concentrations, in which O-Ac groups were eliminated and active amino groups are created, which made it possible for us to replace the original acetyl group with an acyl group. Later on, the polysaccharide is fragmented by means of an oxidation with sodium periodate, which generates carbonyl groups that merge with a protein through a reductive amination, using sodium cyanoborohydride as a reducing agent (US 5425946).

However, it is worthwhile mentioning that this method to replace the N-acetyl groups with N-acyl groups includes the use of a basic hydrolysis at a pH of 13 to 14 (NaOH at 2M), and a temperature range of 90° to 110°, which favors the fragmentation of the polysaccharide and poses the risk of antigenic disturbances. In addition to that, the percentage of N-deacetylation could vary from 30 to 100.

The conjugation methods described so far have included the activation of the PRP in organic solvents, which requires the generation of tetra butyl ammonium salts, the implementation of several purification stages, and the use of several spacers, such as the adipic acid dihydrazine (ADH), the 1.4 di amino butane, or the amino hexanoic acid. Usually, conjugation requires the use of more than one.

Other authors have worked on the conjugation of two polysaccharides and a protein. So is the case for the fusion between the PRP and the Streptococcus pneumoniae type 6a polysaccharide, and the fusion between these and the outer membrane proteins complex of the N. Meningitidis serogroup B (European patent No. 0 534 764 A1).

To obtain this product the following method has been used: first, the PRP should react with oxalic acid and a solution of N-tetra butyl ammonium hydroxide; afterwards, the polysaccharide hydroxyl groups are activated with carbonyl diimidazole in the presence of dimethyl formamide (DMF) and the product obtained at this stage is made to react with the 1.4 di-amino butane and bromoacetyl chloride. Thus, the polysaccharide is activated. The protein is later on activated with the N-Acetyl Homocysteine thiolactone. Afterwards, ditiotreitol (DTT) is added to obtain the sulfhydryl group in the protein that is to react with the activated polysaccharide. In this case, the derivatized PRP and the activated protein are made to react for 18 hours. Thus, a first protein-polysaccharide group is obtained, and here there is a fusion between the SH groups of the protein and the bromo acetyl groups of the polysaccharide. Afterwards, the second polysaccharide is activated (the one obtained from the S. Pneumoniae 6a), for which we first obtain the tetrabutylammonium salt from this polysaccharide by using Dowex50x2 and N-tetra butyl ammonium hydroxide in one column. We then activate the hydroxyl groups with DMF and CDI. They are made to react with the following reagents: 2-(6-aminocapropyl) 4,9-dioxo-1, 12-diaminododecane-naphtalene-1,5-disulphonic acid salt (ACA-DODAD-NDSA) and S-acetyl mercapto succinic anhydride (SAMSA). Thus, the second polysaccharide is derivatized. Next, the second polysaccharide is merged (through the SH group generated) with the aforementioned conjugate by means of the bromo acetyl groups present in the polysaccharide side of the conjugate. The structure of this recently created conjugate will be the following: Polysaccharide 2 - Spacer - Polysaccharide 1 - Spacer - Protein.

Given the number of steps that are necessary to take in this method to activate the antigens, their yield before the conjugation is very low.

As for conjugation, all the methods that exist so far use either the carbonyl groups originated from polysaccharides after their fragmentation, the carboxyl or hydroxyl groups present in the carbon structure of polysaccharides, or the amino and carboxyl groups of proteins. Curiously enough, so far there have not been any reports about the fusion between amino groups generated in the structure of unfragmented polysaccharides and the carboxyl groups of proteins as a strategy for the conjugation of antigenic molecules.

The technical objective pursued with this invention is to create a method to conjugate saccharide antigens by using the generation of amino groups in its structure (without causing major disturbances to its antigenicity) and merge both groups with the carboxyl groups of proteins.

This invention is about a method to conjugate saccharide antigens to obtain multivalent vaccine preparations. It includes the following steps:
a) Treatment of a first saccharide antigen with a basic substance, such as NaOH, KOH, or LiOH, at a concentration of 0.1 to 0.9 N, at a temperature between 40° to 110° C during 3 to 10 hours to favor the generation of amino groups in such a way that the antigenicity of antigens is not damaged;
b) In the case of trivalent preparations, the activation of the carboxyl groups of this first antigen using carbondiimide;
c) Conjugation of the activated carboxyl groups of the antigen obtained after completion of step (b) with a second antigen that could be of protein or saccharide origin;
d) Purification of the conjugate obtained after completion of step (c);
e) Activation of the carboxyl groups of a third protein antigen to increase the immunogenicity of saccharide antigen(s) using carbodiimide;
f) Conjugation of the amino groups generated in the saccharide antigens of step (a) with the activated protein antigen of step (b) to obtain a bivalent preparation. Conjugation of the amino groups present in the saccharide antigens of step (d) with the activated protein antigen of step (e) to obtain a trivalent preparation;
g) Purification of the conjugate obtained after completion of step (f).

This conjugation method would be valid in case the saccharide of step (a) contains within its structure amide-like bonds, given the fact that this saccharide originates from a bacteria. This bacteria could be of the Neisseria gender, particularly the Neisseria meningitidis, of whatever group, A, B, or C. This invention has also considered the possibility when the saccharide mentioned in step (a) originates from a bacteria of the gender Salmonella, particularly the S. Typhi and the polysaccharide is Vi. Likewise, consideration has also been given to the possibility when the saccharide antigen originates from a bacteria of the gender Vibrio, particularly from the species V. Cholerae, specially when the saccharide antigen is the lipopolysaccharide of V. Cholerae.

The method suggested by the present invention would also be valid when the second protein antigen is a peptide or a protein, which could be obtained through natural, recombinant or synthetic procedures, and could come from virus, fungi, plants or animals. This peptide or protein could even come from bacteria, namely: Escherichia coli, Salmonella, Shigella, V. Cholerae, and N. Meningitidis.

Similarly, this method will also be valid when the second antigen originates from a saccharide. This antigen can be obtained from a bacteria of the gender Neisseria, particularly Neisseria Meningitidis, and within it, from either a polysaccharide or a lipopolysaccharide of the groups A, B or C. This invention has also considered the possibility in which the saccharide may come from a bacteria of the gender Salmonella, particularly the S. Typhi, and the polysaccharide is Vi. Likewise this invention has considered a scenario in which the saccharide antigen may come from a bacteria of the gender Vibrio, particularly from the species V. Cholerae, specially when the saccharide antigen is the lipopolysaccharide of V. Cholerae.

The method will also be applicable when the second saccharide antigen comes from H. Influenzae Type B, particularly the Polyribosil Ribitol Phosphate (PRP). The invention will be valid too in case the PRP used as a second saccharide antigen is activated using 1,8 di-amino octane as spacer.

The invention will also be feasible if the third antigen originated from a protein that is used in this method comes from a bacteria. This could be obtained through natural, recombinant, or synthetic procedures, and could come from viruses, fungi, plants, or animals. The antigen could come from a bacteria, namely, N. Meningitidis serogroup B, particularly from proteins of the outer membrane, recombinant proteins (p64K), clostridium tetanic, where the tetanic toxoid, the diphteric toxoid, and the H. Influenzae Type B are all essentially proteins from the outer membrane.

This invention allows for the obtention of conjugate bivalent and trivalent vaccines by means of the method described supra, which requires the implementation of a relatively short number of steps. Besides, conjugates obtained through this method happen to be effective against diseases caused by the bacteria from which the vaccine antigens are obtained.

Also part of the present invention are the vaccine compositions that can be obtained directly through the aforementioned method, namely:
- A vaccine composition where the N. Meningitidis "C" polysaccharide is united, through a covalent bond, to the tetanus toxoid.
- A vaccine composition that contains the N. Meningitidis "C" polysaccharide in conjugation with protein P64k.
- A vaccine composition where the N. Meningitidis "B" lipopolysaccharide (LPS) is united, through a covalent bond, to the tetanus toxoid.
- A vaccine composition that contains the N. Meningitidis "A" polysaccharide in conjugation with the tetanus toxoid.
- A vaccine composition made up by the LPS of V. Cholerae united, through a covalent bond, to the sub-unit B of the toxin of the V. Cholera.
- A vaccine composition where the LPS of the V: cholera is conjugated with the tetanus toxoid.
- Vaccine compositions of conjugates of the polysaccharide Vi of S. Typhi with Shigella proteins, and the polysaccharide Vi. of Styphi is united by means of a covalent bond to proteins of S. Typhi.

Also included in the content of this invention are the trivalent vaccine compositions derived from the use of the aforementioned method. For example, a vaccine composition where the N. Meningitidis "C" polysaccharide is united by a covalent bond to the tetanus toxoid and the PRP; a vaccine composition where the N. Meningitidis "C" polysaccharide is united by a covalent bond to the tetanus toxoid and the N. Meningitidis "A" polysaccharide; the trivalent vaccine composition made up by the polysaccharide Vi of S. Typhi united by a covalent bond to the proteins of Shigella and S. Typhi; the trivalent vaccine composition made up by the polysaccharide Vi. of S. Typhi united by a covalent bond to proteins of Shigella and the LPS of V. Cholera; a vaccine composition where the N. Meningitidis "C" polysaccharide is united by a covalent bond to the tetanus toxoid and the LPS of N. Meningitidis "B"; and the trivalent vaccine composition where the N. Meningitidis "C"polysaccharide is united by a covalent bond to the N. Meningitidis "B" outer membrane proteins and the N. Meningitidis "A" polysaccharide.

The difference that exists between this invention and others that have so far been made is that the conjugate vaccines that are produced through this method are obtained as a result of the generation of amino groups in the saccharide side of the structure, which requires the use of a pH range from 9 to 11 (with the use of NaOH at a concentration of 0.1 to 0.7 N), at a temperature below 110°.

The ranges used have not been reported so far by lierature, and are below the ones referred to by state of the art technologies. Surprisingly, under these conditions, it is possible to attain a high and stable percentage of N-deacetylation. The use of the amino groups generated in the structure of the saccharide to fix the proteins to the saccharide, as described by this method, had not been reported so far. Unexpectedly, a good immunological response against the saccharide side of the conjugate was observed, despite of the fact that there have been authors who had so far advocated for the need to have O-acetyls groups in the structure in order to have a good immunological recognition.

Besides, the spacer that is used in this invention is the bifunctional reagent 1.8 di-amino octane, which had not been reported for this purpose before.

This invention describes a method that could be taken to an industrial scale, since it is a simple method that guarantees high yield and efficient vaccine products.

The demonstration of the following invention is contained in the examples that appear next.

### Example1: Obtention of bivalent vaccines from the Neisseria Meningitidis "C" polysaccharide.

### Example 1.1: N-Deacetylation of the Neisseria Meningitidis "C" polysaccharide.

Sixty (60) milligrams of the group "C" meningococcus polysaccharide were dissolved in 15 ml of NaOH at a concentration of 0.1 to 0.9 N. The solution was placed into the oven at a temperature of 105° C for 5 hours. Afterwards, it was let to cool down at room temperature. The pH was neutralized using hydrochloric acid 1:2 (V/V). The presence of free amino groups in the PMGC was estimated through the technique that uses the ophtaldialdehyde (OPA), the result of which being the generation of amino groups in a step by step series, which increases the concentration of NaOH, as shown in Table 1.

**Tabla 1:**

| Estimation of amino groups generated in the polysaccharide of group "C" meningococos, as a resulto f the N-deacetylation of C-5. | |
|---|---|
| Samples | Amino groups (mmoles/ml) |
| Native Polysaccharide | 0.00 |
| Polysaccharide treated with NaOH 0.1 N | 1.09 |
| Polysaccharide treated with NaOH 0.3 N | 2.64 |
| Polysaccharide treated with NaOH 0.5 N | 4.83 |
| Polysaccharide treated with NaOH 0.7 N | 6.47 |
| Polysaccharide treated with NaOH 0.9 N | 7.85 |

### Example1.2: Coupling of the group NaOH-modified "C" meningococcus polysaccharide with different carrier proteins.

Fifteen milligrams of 1-ethyl-3-(3-dimethylaminopropyl) carbodiimide (EDAC) were added to 5ml of a solution of tetanus toxoid (TT) at a concentration of 1mg/ml (estimated through the Lowry method). The pH was fixed between 4.5 and 6, and the mixture was agitated for 15 minutes at room temperature. Later on 2 ml of the solution of the polysaccharide treated with NaOH, as obtained in the Example 1, were added. The pH was adjusted and the mixture was agitated for 5 hours at room temperature. The mixture was dialyzed throughout the whole night at a temperature of 4° C against 5L of saline phosphate buffer (PBS) at a pH of 7.4. The buffer was changed twice. On the next day the mixture was purified by a column of 16 x 100 mm with Sepharose CL 4B. The buffer used to elute the peak was PBS at a pH of 7.4. The first peak detected was collected at 206 nm, which coincided with the eluted peak at 280 nm. We estimated the protein concentration using the Lowry method, taking as a pattern the bovine seroalbumin (BSA) . The polysaccharide content was estimated using sialic acid, through the resorcinol method. Resulting conjugates were stored at a temperature of 4° C. Thimerosal (0.01 per cent) was added to the sample until its further use.

In the case of proteins P64K (recombinant) and VME, both from N. Meningitidis serogroup "B", the same procedure described for the TT was followed. Table 2 shows the results.

**Tabla 2:**

| Results of the conjugate process | | | |
|---|---|---|---|
| Conjugate | Protein(mg/ml) | Sialic Acid (mg/ml) | Mole-protein ratio PMGC |
| PMGC - TT | 0.1588 | 0.3496 | 3:1 |
| PMGC - P64K | 0.1854 | 0.1535 | 2:1 |
| PMGC - VME | 0.1695 | 0.2536 | 3.3:1 |
| PMGC: group C meningococcus polysaccharide | | | |

### Example 1.3: Immunization procedure

Groups of 8 white male Balb/c mice (8-10 weeks old) were intraperitoneally inoculated with 3 doses (0, 14, and 28 days; each dose contained a polysaccharide concentration of 10ug) of each one of the conjugates prepared as indicated in the Example 2. Before each inoculation and 7 and 14 days after the last inoculation, we extracted blood from the animals in order to obtain the sera that would be further evaluated in clinical trials to corroborate the existence of antibodies and bactericides.

### Example 1.4: Estimation of IgG antibodies in mice through the ELISA method.

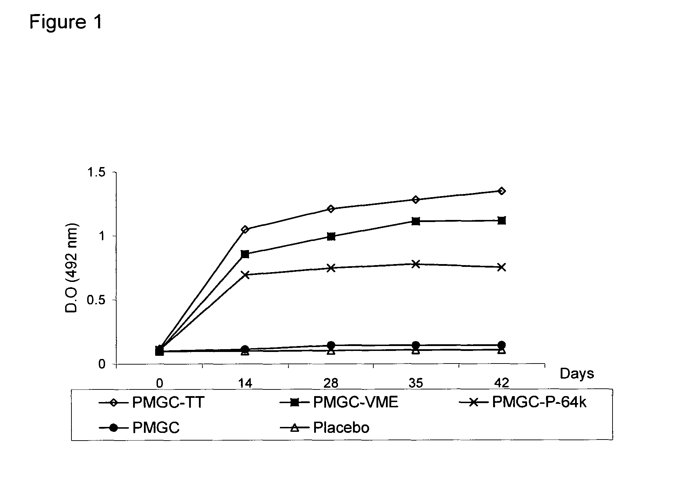

This technique was applied with the use of Maxisorp plates (Nunc) with a poly-L lysin coating (100 :I / well) and was left to incubate for 1 hour at room temperature in a humid chamber. After that, the plate was washed four times with PBS, pH 7.4, plus 0.5 ml of Tween-20 dissolved in one liter of buffer solution. It was then coated with a solution of PMGC (5 :I) or VME (depending on what the interest may be), with an addition of 100 :I / well, and it was left to incubate for 4 hours in a humid chamber at a temperature of 4° C. The plate was then washed four times with PBS (pH 7.4) and 0.5 ml of Tween-20 dissolved in one liter of buffer solution. It was then coated with a solution of glutaraldehyde (in PBS at 0.015 per cent), with an addition of 100 :I / well. Then it was left to incubate for 1 hour in a humid chamber at a temperature of 37° C. The plate was then washed four times with PBS (pH 7.4) and 0.5 ml of Tween-20 dissolved in one liter of buffer solution and then it was coated with a gel solution (100 :I / ml) with an addition of 100 :I / well. It was left to incubate for 1 hour in a humid chamber at a temperature of 37° C. The plate was then washed four times and, as established by the protocol, we added 100 :I / well of serum (a dilution of 1 / 800) from each sample. It was left to incubate for 1 hour in a humid chamber at a temperature of 37° C. The plate was then washed four times. We added 100 :I / well of the second antibody, which consisted of one mice anti-IgG produced in a ram, conjugated to peroxidase (Sigma No. 3742). It was left to rest for 1 hour at a temperature of 37° C. Later on, after washing the plate we added the substrate 1,2-phenylendiamin plus hydrogen peroxide (OPD + H₂O₂). It was left to rest for 15 minutes at room temperature, and the reaction was stopped by adding 50 :I / well of sulphuric acid 2N. The DO values were read at 49 nm on a Titertek Multieskam equipment. Results are shown on Figure 1.

### Example 1.5: Estimation of subclasses of IgG antibodies in mice using the ELISA method.

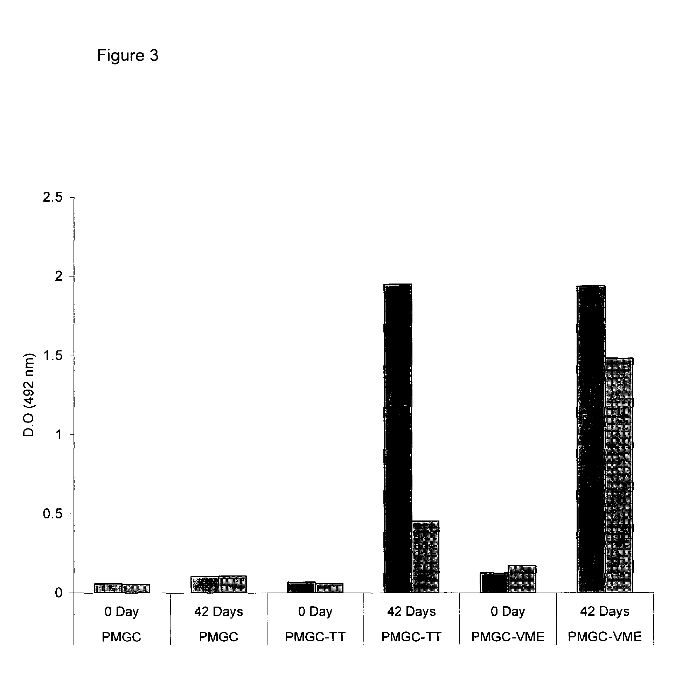

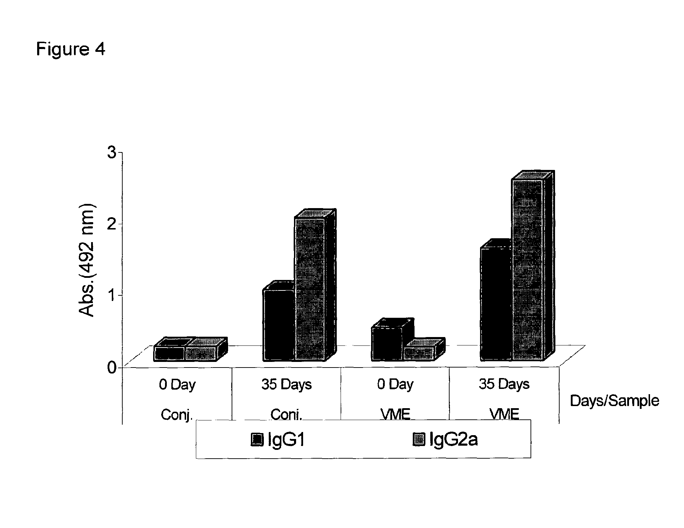

In order to estimate the IgG subclasses present in animal serum we used a biotin-streptavidin amplification ELISA. We used 96 polystyrene plates of flat bottom well (Maxisorp, Nunc), which were coated with poly-L-lysin at a concentration of 3 :I / ml diluted in PBS. Plates were incubated for 30 minutes at room temperature, and after that time they were washed three times with PBS. A second coating was added using PMGC or VME (depending on what the interest may be). They were left to incubate at a temperature of 4° C during the whole night and then they were blocked with PBS + BSA + Tween 20. The samples used were prepared using a 1:100 dilution in PBS/Tween 20 with BSA. They were left to incubate during the whole evening at a temperature of 4° C. After that we added the conjugates anti IgG1 or biotilinated IgG 2^{a} (Sigma). Afterwards, we added streptavidin (Sigma). As a substrate we used a 100 :I / bowl of H₂O₂ 0.15 % with O-phenylendiamine (Sigma) and the reaction was stopped with H₂SO₄, 2.5 N. Absorbency was fixed at 492 nm by means of a micro-ELISA reader (Titertek Multieskan ®). At each an every step of the incubation, samples were washed with PBS/Tween 20. Figures 3 and 4 show the results of these estimations.

### Example 1.6: Estimation of bactericidal antibodies.

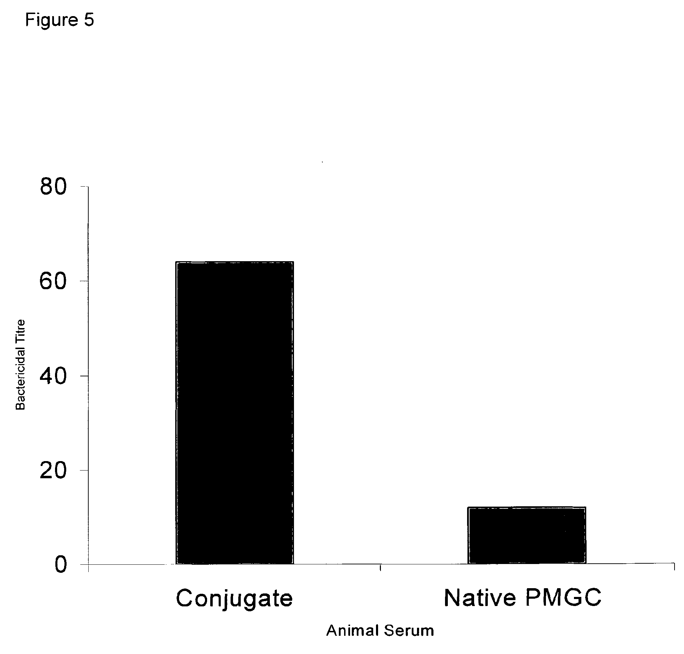

It is known that bactericidal antibodies play a very important role in the protection of vaccinated individuals. Vaccines that have been manufactured using polysaccharides should be able to generate bactericidal antibodies in order to ensure proper protection. That is the reason why we felt encouraged to apply this technique to the serum of vaccinated mice. Results are shown in Figure 5.

The results described in this work evidence the creation of the first Cuban conjugated vaccine against N. Meningitidis serogroup C, which at the same time, is the first conjugated vaccine in the world using the amino groups generated by the polysaccharide chain (as a result of the elimination of acetyls groups fused with NH that are present in the polysaccharide chain) to create a covalent bond with proteins, which happens to be highly immunogenic not only against N. Meningitidis serogroup C, but also against carrier proteins (N. Meningitidis serogroup B or tetanus toxoid, depending on the formula used). This vaccine will protect children below five years of age against diseases caused by these bacteria.

### Example 2: Creation of bivalent vaccines using V. Cholera lipopolysaccharide.

### Example 2.1: N-deacetylation of V. Cholera lipopolysaccharide.

We dissolved 30 mg of the LPS into 8 ml of NaOH at concentrations ranging between 0.25 to 1 N. We place the mixture into the oven at a temperature of 105° C for 2, 4, and 6 hours. Afterwards we let it cool at room temperature. The pH was neutralized using hydrochloric acid 1:2 (V/V). The presence of free amino groups in the LPS was estimated using the OPA technique. The result of this procedure was the gradual generation of amino groups as long as the NaOH concentration increases. The longer the time of reaction, the higher the concentration. Results are shown in Figure 3.

**Table 3:**

| Estimation of amino groups generated in the LPS as a result of the N de-acetylation of fatty acids contained in Lipid A. | | |
|---|---|---|
| Samples | Time(h ) | NH₂ Groups (mmoles/mg) |
| Native LPS | - | 0.61 |
| LPS treated with NaOH 0.25 N | 2 | 0.9 |
| LPS treated with NaOH 0.25 N | 4 | 1.05 |
| LPS treated with NaOH 0.25 N | 6 | 1.27 |
| LPS treated with NaOH 0.5 N | 2 | 1.15 |
| LPS treated with NaOH 0.5 N | 4 | 1.57 |
| LPS treated with NaOH 0.5 N | 6 | 1.96 |
| LPS treated with NaOH 1 N | 2 | 1.70 |
| LPS treated with NaOH 1 N | 4 | 1.94 |
| LPS treated with NaOH 1 N | 6 | 2.16 |

### Example 2.2: Coupling of the NaOH- modified Vibrio cholerae lipopolysaccharide with the tetanus toxoid as a carrier protein.

We used 3ml of a tetanus toxoid solution at a concentration of 1mg/ml (estimated according to the Lowry method). We added 15 mg of EDAC. The pH was fixed between 4.5 and 6. It was then stirred at room temperature for 15 minutes. Later on we added 2 ml of the NaOH-treated LPS solution, which was obtained as described in the Example 1. The pH was fixed at 8. Then it was let to stir for 5 hours at room temperature. Afterwards it was let to dialyze during the whole night at a temperature of 4° C against 5 L of PBS at a pH of 7.4. The buffer was changed twice. The next day it was purified by a 16 x 100 mm column with Sepharose CL 4B. The buffer used to elute the peak was PBS with a pH of 7.4. We collected he first peak detected at 206 nm which coincided with the eluted peak at 280 nm. We estimated the protein concentration using the Lowry method. The pattern used was the BSA and the polysaccharide content, which was obtained through the phenol-sulphuric method. Resulting conjugates were stored at a temperature of 4° C. We added 0.01 per cent of thimerosal until it was eventually used. The results obtained are shown in Table 4.

**Table 4:**

| Results of the conjugation process. | | | |
|---|---|---|---|
| Conjugate | LPS (mg/ml) | Protein (mg/ml) | LPS-Protein Ratio |
| LPS (1 N) - TT | 0.428 | 0.410 | 1:1 |

### Example 2.3: Estimation of toxicity using the Limulus Amebocytes Lysate method (LAL).

The lipopolysaccharide toxicity was estimated using the Limulus Amebocytes Lysate test (LAL), through the chromogenic method, with the kit Coatest Endotoxin®, of the Swedish Company Chromogenix AB. The test was run following the technique created by the manufacturer, using as a pattern a curb between 0.15 and 1.2 endotoxin units (EU). We chose to work with essay tubes, including a dry block heater "Stuart Scientific" for 24 tubes. In order to apply this method we used 10 x 75 mm borosilicate reaction tubes (Pyrotubes®) manufactured by the company "Associates of Cape Cod". We also used "Costar" polystyrene 15 ml centrifuge tubes for the pattern curb and "Rainin" pipettes. All these materials were free from endotoxins.

As a result of the LAL test the native LPS registered a value of 5.3477 EU/ng, while the LPS treated under the conditions described in the example 1 showed values of 0.001 EU/ng, which represents a reduction of the original toxicity by more than 5000 times.

### Example 2.4: The use of the ELISA method to estimate the antigenicity of he V. Cholera LPS after conjugation.

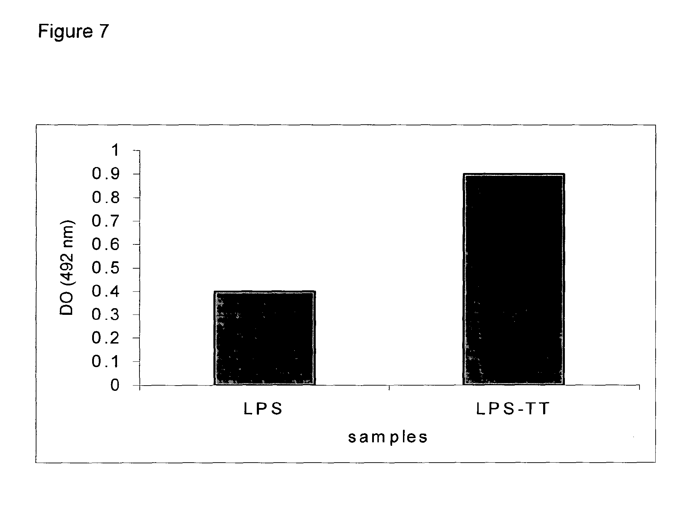

In order to check whether or not after the hydrolysis and conjugation process the LPS preserved the original epitopes, we performed an inhibition ELISA test using Maxisorp plates (Nunc). We used as a coating antigen the conjugated obtained from the LPS treated with 1 N of NaOH after four hours at a temperature of 105° C; the native LPS as control; a human anti IgG-HRP conjugate diluted at 1/4000; and as a developer we used the SIGMA's o-phenyldiamine (OPD). The antibody used was the serum obtained from humans who had been orally inoculated with an attenuated strain of V. Cholerae O1 EI Tor Ogawa, an oral vaccine candidate against cholera. The results obtained are shown in Figure 7.

### Example 2.5: Immunization procedure

Groups of 8 Balb/c white male mice (8 to 10 weeks old) were intraperitoneally inoculated with 3 dose of each of the conjugates prepared in the Example 2 (0, 7, and 28 days; each dose contained a concentration of 10 ug LPS). Previous to every inoculation, and 7 days after the last inoculation we extracted some blood from the animals in order to obtained the serum that was to be evaluated in the immunochemical tests.

### Example 2.6: Estimation of IgG antibodies in mice using the ELISA method.

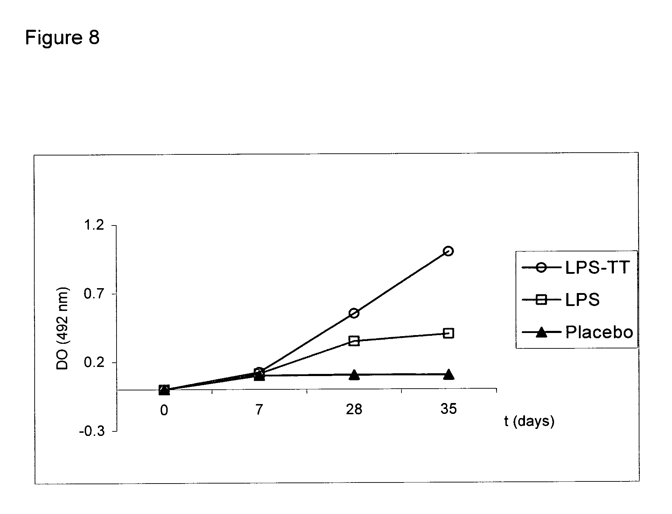

This technique was applied using Maxisorp plates (Nunc) with a poly-L- lysin coating (100 :I / well) followed by 1 hour incubation at room temperature in a humid chamber. Afterwards we washed the plate four times with PBS at a pH of 7.4, plus 0.5 ml of Tween 20 per liter of buffer, and we coated it with a solution of LPS (5 :I /ml) or protein (depending on what the interest may be). We added 100 :I / well and we left it to incubate for 4 hours in a humid chamber at a temperature of 4° C. We washed the plate four times with PBS at a pH of 7.4, plus 0.5 ml of Tween 20 per liter of buffer, and we coated it with a solution of glutaraldehyde (in PBS at 0.015 per cent). We added 100 :I / well and we left it to incubate for 1 hour in a humid chamber at a temperature of 37° C. We washed the plate four times with PBS at a pH of 7.4, plus 0.5 ml of Tween 20 per liter of buffer, and we coated it with a gel solution (100 :I /ml). We added 100 :I / well and we left it to incubate for 1 hour in a humid chamber at a temperature of 37° C. We washed the plate four times and, as established by the protocol, we added 100 :I / well of the serum of each sample (dilution = 1/800) and we left it to incubate for 1 hour in a humid chamber at a temperature of 37° C. We washed the plate four times and we added 100 :I / well of the second antibody, which consisted of one mice anti-IgG produced in ram which was conjugated to peroxidase (Sigma No. 3742). It was let to rest for 1 hour at a temperature of 37° C. After the plate was washed, we added the substrate (OPD + H₂O₂), we waited for 15 more minutes while the plate remained at room temperature, and we stopped the reaction by adding 50 :I / well of 2N sulphuric acid. DO values were read at 492 nm in a Titertek Multieskam equipment. Results are shown in Figure 8.

### Example 3: Obtention of bivalent vaccines from the S. Typhi Vi polysaccharide.

### Example 3.1: N-deacetylation of S. Typhi Vi polysaccharide.

We dissolved 20 mg of Vi polysaccharide in 5 ml of NaOH. We placed the mixture into the oven at a temperature that ranged between 50° C to 60° C during a period of time that ranged between 8 to 12 hours. Afterwards, we let the mixture to cool down at room temperature and we neutralized the pH with 1:2 (V/V) hydrochloric acid. The presence of free amino groups in the Vi polysaccharide was estimated by applying the OPA technique, the result of which was the generation of 1.2 :mols of amino groups per ml of solution.

### Example 3.2: Coupling of the NaOH-modified Vi polysaccharide with different carrier proteins.

We added 15 mg of EDAC to 2ml of tetanus toxoid (TT) solution with a concentration of 1 mg/ml. We fixed the pH between 4.5 and 6. It was stirred for 15 minutes at room temperature. Afterwards we added 2ml of the activated Vi polysaccharide solution, as described by the Example 3.1. We adjusted the pH and stirred it for 5 hours at room temperature. It was then dialyzed during the whole night at a temperature of 4° C against 5L of PBS with a pH of 7.4. We changed the buffer twice. The day after it was submitted to a chromatography, using a 16 x 100 mm column with Sepharose CL 4B. The buffer used to elute the peak was PBS at a pH of 7.4. We collected the first peak detected at 206 nm, which coincided with the peak elute at 280 nm, at which we estimated the protein concentration through the Lowry method, using as a pattern the BSA. The polysaccharide content was estimated by an ELISA method which was put in place for this purpose. Resulting conjugates were stored at a temperature of 4° C,. With an additional 0.01 per cent of thimerosal up until it was eventually used.

In the case of the VME protein of S. Typhi and the VME proteins of serogroup B N. Meningitidis we followed the same procedure described for the TT. Results are shown in the following table.

**Tabla 5:**

| Resultados del proceso de conjugación | | | |
|---|---|---|---|
| Conjugate | Vi (mg/ml) | Protein (mg/ml) | Vi/Protein Ratio |
| Vi-VMEs | 0.51 | 0.49 | 1/1 |
| Vi-VMEm | 0.47 | 0.43 | 1.1/1 |
| Vi - TT | 0.48 | 0.46 | 1/1 |
| VMEs= Vesicle of the outer membrana of Salmonella Typhi | | | |
| VMEm= Vesicle of the outer membrana of serogroup B N. Meningitidis | | | |

### Example 3.3: Immunization procedure

Groups of 8 Balb/c white male mice (8 to 10 weeks old) were intraperitoneally inoculated with 2 dose of each of the conjugates prepared as described in the Example 2 (0, and 28 days; each dose contained a concentration of 10 ug of Vi). Previous to every inoculation, and 14 days after the last inoculation we extracted some blood from the animals in order to obtained the serum that was to be evaluated in the immunochemical tests.

### Example 3.4: Estimation of IgG antibodies in mice using the ELISA method.

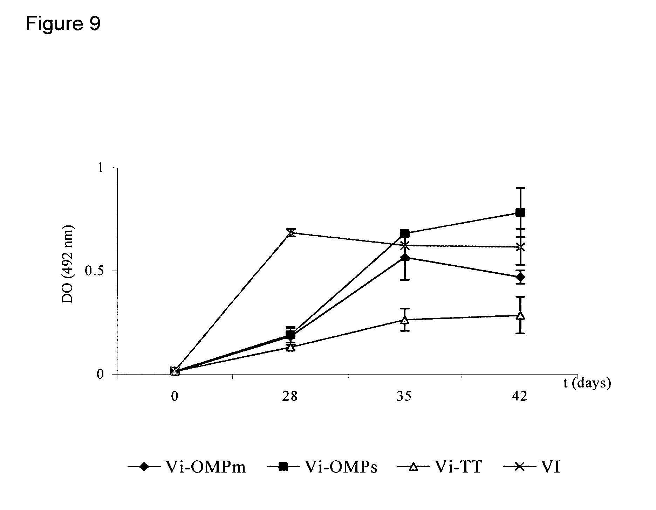

This technique was applied with the use of Maxisorp plates (Nunc) with a poly-L lysin coating (100 :I / well) and was left to incubate for 1 hour at room temperature in a humid chamber. After that, the plate was washed four times with PBS, pH 7.4, plus 0.5 ml of Tween-20 dissolved in one liter of buffer solution. It was then coated with a solution of Vi polysaccharide (5 :g/ ml) or protein (depending on what the interest may be), with an addition of 100 :I / well, and it was left to incubate for 4 hours in a humid chamber at a temperature of 4° C. The plate was then washed four times with PBS (pH 7.4) and 0.5 ml of Tween-20 dissolved in one liter of buffer solution. It was then coated with a solution of glutaraldehyde (in PBS at 0.015 per cent), with an addition of 100 :I / well. Then it was left to incubate for 1 hour in a humid chamber at a temperature of 37° C. The plate was then washed four times with PBS (pH 7.4) and 0.5 ml of Tween-20 dissolved in one liter of buffer solution and then it was coated with a gel solution (100 :g / ml) with an addition of 100 :I / well. It was left to incubate for 1 hour in a humid chamber at a temperature of 37° C. The plate was then washed four times and, as established by the protocol, we added 100 :I / well of serum (a dilution of 1 / 800) from each sample. It was left to incubate for 1 hour in a humid chamber at a temperature of 37° C. The plate was then washed four times. We added 100 :I / well of the second antibody, which consisted of one mice anti-IgG produced in a ram, conjugated to peroxidase (Sigma No. 3742). It was left to rest for 1 hour at a temperature of 37° C. Later on, after the plate was washed we added the substrate (OPD + H₂O₂). It was left to rest for 15 minutes at room temperature, and the reaction was stopped by adding 50 :I / well of 2N sulphuric acid. The DO values were read at 492 nm on a Titertek Multieskam equipment. Results are shown on Figure 9.

### Example 4: Obtention of trivalent vaccines from the Neisseria Meningitidis C polysaccharide and the Haemophilus ifluenzae polysaccharide.

### Example 4.1: The N-deacetylation of the Neisseria Meningitidis C polysaccharide.

Sixty (60) milligrams of the group "C" meningococcus polysaccharide were dissolved into 15 ml of NaOH. The solution was placed into the oven during a period of time that ranged between 3 to 4.5. Afterwards, it was let to cool down at room temperature. The pH was neutralized using 1:2 (V/V) hydrochloric acid. The presence of free amino groups in the group "C" meningococcus polysaccharide was estimated through the OPA technique, the result being the generation of 6.7 mM amino groups.

### Example 4.2: Activation of hydroxyl groups of the Type "B" H. Influenzae polysaccharide (PRP).

We dissolved 40 mg of PRP into 300 ul of distilled water. We added this to a solution of 1 ml of CDI dissolved into dimethylsulphoxide (DMSO). It was stirred for 3 hours. Afterwards, we prepared a solution containing 70 mg of 1, 8 diaminooctane dissolved into 0.7 ml of distilled water and NaOH 5 N. It was stirred in cold bain-marie for 60 minutes. After that, we added the PRP-CDI to the spacer and allowed the reaction to continue for 1 hour in a cold bain-marie. We then let it t rest for 20 minutes at room temperature. Finally, we dialyzed the mixture at a temperature of 4° C against 5L of PBS with a pH of 7.4. We changed the buffer 6 times, and when that process concluded, we took the mixture out from the dialysis pouch and stored it at a temperature of 4° C.

We checked the presence of amino groups in this activated PRP by using the OPA technique, which resulted into a concentration of 4.4 mmols of NH₂/mg of PRP.

### Example 4.3: Coupling of the modified group "C" meningococcus polysaccharide with the the activated PRP.

The solution consisting of 20 mg of PMGC and 3 ml of PBS with a pH of 7.4 which was modified in the Example 4.1, was added 30 mg of EDAC. It was stirred for 25 minutes at room temperature. After that we added the activated PRP of the Example 4.2. The mixture was then stirred for 3 hours and diafiltered against PBS at a pH of 7.4. Diafiltration was repeated 5 times against 50 ml of buffer every time. After the last diafiltration, it was left in 2 ml and was stored at a temperature of 4° C until it was eventually used.

### Example 4.4: Coupling of conjugated polysaccharides with carrier proteins.

We added 50 mg of EDAC to 10 ml of a tetanus toxoid (TT) solution with a concentration of 1mg / ml (estimated according to the Lowry method). It was stirred for 15 minutes at room temperature. Afterwards we added 2ml of the polysaccharide solution as obtained in the Example 4.3. Then it was stirred for 2 to 4 hours at room temperature. It was then dialyzed during the whole night at a temperature of 4° C against PBS with a pH of 7.4 (5L). We changed the buffer twice. The day after it was purified using a 16 x 100 mm column with Sepharose CL 4B. The buffer used to elute the peak was PBS at a pH of 7.4. We collected the first peak detected at 280 nm, at which we estimated the protein concentration through the Lowry method, using as a pattern the BSA. The PMGC content per sialic acid was estimated by the resorcinol method. The PRP content determining ribose was estimated by the orcinol method. Resulting conjugates were stored at a temperature of 4° C, with an additional 0.02 per cent of thimerosal up until it was eventually used.

### Example 4.5: Immunization procedure

Groups of white male Balb/c mice (8-10 weeks old) were intraperitoneally inoculated with 3 dose (0, 14 and 28 days; each dose contained a polysaccharide concentration of 10ug of PMGC) of the conjugate as prepared in Example 4. Twelve days after the last inoculation, we extracted blood from the animals in order to obtain the sera that would be further evaluated by the immunogenicity and bactericidal tests.

### Example 4.6: Estimation of antibodies in mice using the ELISA method.

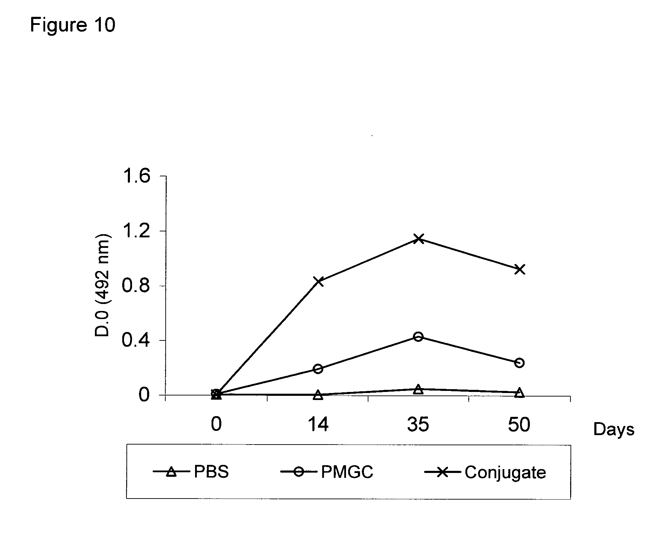

This technique was applied using Maxisorp plates (Nunc) with a poly-L- lysin coating (100 :I / well) followed by 1 hour incubation at room temperature in a humid chamber. Afterwards the plate was washed four times with PBS at a pH of 7.4, plus 0.5 ml of Tween 20 per liter of buffer, and we coated it with a solution of PMGC or PRP (5 :g /ml). We added 100 :I / well and we let it incubate for 4 hours in a humid chamber at a temperature of 4° C. Then the plate was washed four times with PBS at a pH of 7.4, plus 0.5 ml of Tween 20 per liter of buffer, and was coated with a solution of glutaraldehyde (in PBS at 0.015 per cent). We added 100 :I / well and we let it incubate for 1 hour in a humid chamber at a temperature of 37° C. The plate was washed four times with PBS at a pH of 7.4, plus 0.5 ml of Tween 20 per liter of buffer, and was coated with a gel solution (100 :g /ml). We added 100 :I / well and we let it incubate for 1 hour in a humid chamber at a temperature of 37° C. The plate was washed four times and, as established by the protocol, we added 100 :I / well of the serum of each sample. We let it incubate for 1 hour in a humid chamber at a temperature of 37° C. The plate was washed four times. We added 100 :I / well of the second antibody, which consisted of one mice anti-IgG produced in ram that was conjugated to peroxidase (Sigma No. 3742). It was let to rest for 1 hour at a temperature of 37° C. After the plate was washed, we added the substrate (OPD + H₂O₂), we waited for 15 more minutes while the plate remained at room temperature, and we stopped the reaction by adding 50 :I / well of 2N sulphuric acid. DO values were read at 492 nm in a Titertek Multiskam® equipment. Absorbency values are shown in Figures 10, 11, and 12.

### Example 4.7: Estimation of subclasses of IgG antibodies in mice using the ELISA method.

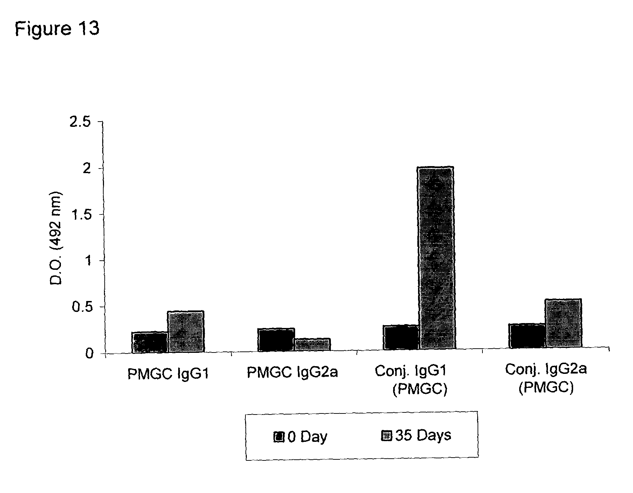

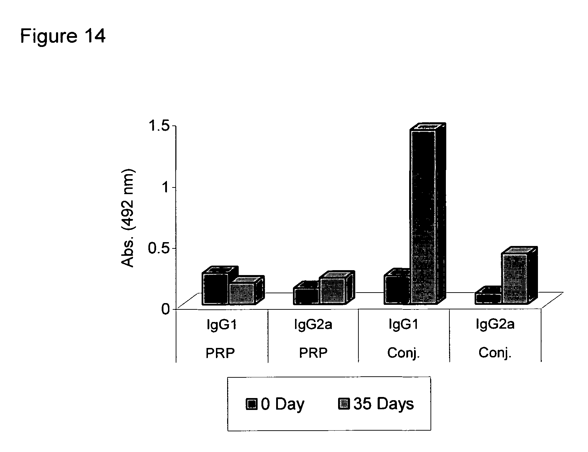

In order to estimate the IgG subclasses present in animal serum we used a biotin-streptavidin amplification ELISA test. We used 96 well flat-bottom polystyrene plates (Maxisorp, Nunc), which were coated with poly-L-lysin at a concentration of 3 :g / ml diluted in PBS. Plates were incubated for 30 minutes at room temperature, and after that time they were washed three times with PBS. A second coating was applied using the aforementioned polysaccharide (Poly C or PRP). They were let to incubate at a temperature of 4° C during the whole night and then they were blocked with PBS + BSA + Tween 20. The samples used were prepared using a 1:100 dilution in PBS / Tween 20 with BSA. They were let to incubate during the whole night at a temperature of 4° C. After that we added the anti IgG1 and the biotilinated IgG 2a conjugates (Sigma). Then we added streptavidin (Sigma). As a substrate we used a 100 :I / bowl of H₂O₂ 0.15 % with O-phenylendiamine (Sigma) and the reaction was stopped with H₂SO₄, 2.5 N. Absorbency was measured at 492 nm by means of a micro-ELISA reader (Titertek Multieskan). After each step of incubation, samples were washed with PBS/Tween 20. Results are shown in Figures 13 and 14.

### Example 5: Obtention of bivalent vaccines from serogroup "A" Neisseria Meningitidis polysaccharide.

### Example 5.1: N-Deacetylation of serogroup "A" Neisseria Meningitidis polysaccharide.

Thirty (30) milligrams of group "A" meningococcus polysaccharide (PMGA) were dissolved in 8 ml of NaOH at a concentration range of 0.5 to 0.9 N. The solution was placed into the oven at a temperature range of 40° C to 60° C during a period of time that fluctuated between 1 and 8 hours. Afterwards, it was let to cool down at room temperature. The pH was neutralized with 1:2 (V/V) hydrochloric acid. The presence of free amino groups in the PMGA was estimated through the (OPA) technique, the result of which being the generation of 2.5 µmols of amino groups per ml of solution.

### Example 5.2: Coupling of the NaOH-modified group "A" meningococcus polysaccharide with the tetanus toxoid.

We used 3ml of a tetanus toxoid (TT) solution at a concentration of 1 mg / ml (estimated according to the Lowry method). We added 15 mg of EDAC. The pH was fixed between 4.5 and 6. It was then stirred at room temperature for 15 minutes. Later on we added 2 ml of the activated PMGA solution, as obtained in the Example 5.1. The pH was adjusted . Then it was stirred for 5 hours at room temperature. Afterwards it was let to dialyze during the whole night at a temperature of 4° C against 5 L of PBS at a pH of 7.4. The buffer was changed twice. The next day it was purified by a 16 x 100 mm column with Sepharose CL 4B. The buffer used to elute the peak was PBS with a pH of 7.4. We collected he first peak detected at 206 nm, which coincided with the eluted peak at 280 nm. At this point we estimated the protein concentration using the Lowry method. The pattern used was the BSA and the polysaccharide content, through the estimation of phosphate. Resulting conjugates were stored at a temperature of 4° C. We added 0.01 per cent of thimerosal until it was eventually used. The results obtained during the conjugation process are shown in Table 6.

**Tabla 6:**

| Resultados del proceso de conjugación. | | | |
|---|---|---|---|
| Conjugate | PMGA (mg/ml) | Protein (mg/ml) | PMGA / Protein ratio |
| PMGA - TT | 0.91 | 0.89 | 1:1 |
| PMGA = group "A" meningococcus polysaccharide. | | | |

### Example 5.3: Immunization procedure

Groups of 8 white male Balb/c mice (8-10 weeks old) were intraperitoneally inoculated with 2 dose (0, and 28 days; each dose contained a concentration of 10ug of PMGA) of each of the conjugates prepared as described by the Example 2. Previous to every inoculation and 14 days after the last inoculation, we extracted blood from the animals in order to obtain the sera that would be further submitted to immunochemical tests.

### Example 5.4: Estimation of IgG antibodies in mice through the ELISA method.

This technique was applied with the use of Maxisorp plates (Nunc) with a poly-L lysin coating (100 :I / well) and was left to incubate for 1 hour at room temperature in a humid chamber. After that, the plate was washed four times with PBS, pH 7.4, plus 0.5 ml of Tween-20 dissolved in one liter of buffer solution. It was then coated with a solution of PMGA (5 :g / ml) or TT (depending on what the interest may be), with the addition of 100 :I / well, and we let it to incubate for 4 hours in a humid chamber at a temperature of 4° C. The plate was then washed four times with PBS (pH 7.4) and 0.5 ml of Tween-20 dissolved in one liter of buffer solution. It was then coated with a solution of glutaraldehyde (in PBS at 0.015 per cent), with the addition of 100 :I / well. Then it was let to incubate for 1 hour in a humid chamber at a temperature of 37° C. The plate was then washed four times with PBS (pH 7.4) plus 0.5 ml of Tween-20 dissolved in one liter of buffer solution and then it was coated with a gel solution (100 :g / ml) with the addition of 100 :I / well. It was let to incubate for 1 hour in a humid chamber at a temperature of 37° C. The plate was then washed four times and, as established by the protocol, we added 100 :I / well of serum (a dilution of 1 / 800) from each sample. It was let to incubate for 1 hour in a humid chamber at a temperature of 37° C. The plate was then washed four times. We added 100 :I / well of the second antibody, which consisted of one mice anti-IgG produced in ram, conjugated to peroxidase (Sigma No. 3742). It was let to rest for 1 hour at a temperature of 37° C. Later on, after washing the plate, we added the substrate (OPD + H₂O₂). It was let to rest for 15 minutes at room temperature, and the reaction was stopped by adding 50 :I / well of 2N sulphuric acid. The DO values were read at 492 nm on a Titertek Multieskam equipment.

### Example 6: Obtention of bivalent vaccines from serogroup "B" Neisseria Meningitidis polysaccharide.

### Example 6.1: N-Deacetylation of serogroup "B" Neisseria Meningitidis polysaccharide.

Twenty (20) milligrams of the LPS were dissolved in 5 ml of NaOH. The solution was placed into the oven at a temperature range of 90° C to 105° C during a period of time that fluctuated between 2 and 4 hours. Afterwards, it was let to cool down at room temperature. The pH was neutralized with 1:2 (VN) hydrochloric acid. The presence of free amino groups in the LPS was estimated through the OPA technique, the result of which being the generation of amino groups as shown in Table 7.

**Table 7:**

| Estimation of LPS-generated amino groups as a results of the N-Deacetylation of Lipid "A" fatty acids. | | |
|---|---|---|
| Samples | Time (hours) | NH₂ Groups (mmols / mg) |
| Native LPS | - | 0.61 |
| LPS treated with NaOH | 4 | 2.4 |

### Example 6.2: Coupling of the NaOH-modified serogroup "B" Neisseria Meningitidis polysaccharide with the tetanus toxoid as a carrier protein.

We used 2ml of a tetanus toxoid (TT) solution at a concentration of 1 mg / ml (estimated according to the Lowry method). We added 15 mg of EDAC. The pH was adjusted between 4.5 and 6. It was then stirred at room temperature for 15 minutes. Later on we added 2 ml of the LPS solution treated with NaOH, as obtained in the Example 1. The pH was fixed at 8. Then it was stirred for 5 hours at room temperature. Afterwards it was let to dialyze during the whole night at a temperature of 4° C against 5 L of PBS at a pH of 7.4. The buffer was changed twice. The next day it was purified by a 16 × 100 mm column with Sepharose CL 4B. The buffer used to elute the peak was PBS with a pH of 7.4. We collected he first peak detected at 206 nm, which coincided with the eluted peak at 280 nm. At this point we estimated the protein concentration through the Lowry method, using the BSA as a pattern. The polysaccharide content was estimated using the phenol-sulphuric method. Resulting conjugates were stored at a temperature of 4° C. We added 0.01 per cent of thimerosal until they were eventually used. The results obtained are shown in Table 8.

**Table 8:**

| Results of the conjugation process. | | | |
|---|---|---|---|
| Conjugate | LPS (mg/ml) | Protein (mg/ml) | LPS : Protein ratio |
| LPS(0.7N) - TT | 1.0 | 1.22 | 0.8:1 |

### Example 6.3: Estimation of toxicity using the Limulus Amebocytes Lysate method (LAL).

The lipopolysaccharide toxicity was estimated using the Limulus Amebocytes Lysate test (LAL), through the chromogenic method, with the Coatest Endotoxin® kit of the Swedish Company Chromogenix AB. The test was run following the technique created by the manufacturer, using as a pattern a curb between 0.15 and 1.2 endotoxin units (EU). We chose to work with essay tubes, including a dry block heater "Stuart Scientific" for 24 tubes. In order to apply this method we used 10 x 75 mm borosilicate reaction tubes (Pyrotubes®) manufactured by the company "Associates of Cape Cod". We also used "Costar" polystyrene 15 ml centrifuge tubes for the pattern curb and "Rainin" pipettes. All these materials were free from endotoxins.

As a result of the LAL test we found out that the native LPS registered a value of 12140 EU / µg, while the LPS treated under the conditions described in the Example 1 showed values of 5.0 EU / µg, which represents a reduction of the original toxicity by more than 2000 times.

### Example 6.4: Immunization Procedure

Groups of 8 white male Balb/c mice (8-10 weeks old) were intraperitoneally inoculated with 2 dose (0, and 28 days; each dose contained a polysaccharide concentration of 10ug) of each one of the conjugates prepared as described by the Example 2. Before each inoculation and 14 days after the last inoculation, we extracted blood from the animals in order to obtain the sera that would be further submitted to immunochemical tests.

### Example 6.5: Estimation of IgG antibodies in mice through the ELISA method.

This technique was applied with the use of Maxisorp plates (Nunc) with a poly-L lysin coating (100 :I / well) and was left to incubate for 1 hour at room temperature in a humid chamber. After that, the plate was washed four times with PBS, pH 7.4, plus 0.5 ml of Tween-20 dissolved in one liter of buffer solution. It was then coated with a solution of LPS (5 :g / ml) or TT (depending on what the interest may be), with an addition of 100 :I / well, and it was let to incubate for 4 hours in a humid chamber at a temperature of 4° C. The plate was then washed four times with PBS (pH 7.4) and 0.5 ml of Tween-20 dissolved in one liter of buffer solution. It was then coated with a solution of glutaraldehyde (in PBS at 0.015 per cent), with an addition of 100 :I / well. Then it was let to incubate for 1 hour in a humid chamber at a temperature of 37° C. The plate was then washed four times with PBS (pH 7.4) and 0.5 ml of Tween-20 dissolved in one liter of buffer solution and then it was coated with a gel solution (100 :g / ml) with an addition of 100 :I / well. It was let to incubate for 1 hour in a humid chamber at a temperature of 37° C. The plate was then washed four times and, as established by the protocol, we added 100 :I / well of serum (a dilution of 1 / 800) from each sample. It was let to incubate for 1 hour in a humid chamber at a temperature of 37° C. The plate was then washed four times. We added 100 :I / well of the second antibody, which consisted of one mice anti-IgG produced in ram, conjugated to peroxidase (Sigma No. 3742). It was let to rest for 1 hour at a temperature of 37° C. Later on, after washing the plate we added the substrate (OPD + H₂O₂). It was let to rest for 15 minutes at room temperature, and the reaction was stopped by adding 50 :I / well of 2N sulphuric acid. The DO values were read at 492 nm on a Titertek Multieskam equipment.

### Example 7: Obtention of trivalent vaccines from the Neisseria Meningitidis "C" polysaccharide and the Neisseria Meningitidis "A" polysaccharide.

### Example 7.1: N-deacetylation of the Neisseria Meningitidis "C" polysaccharide.

Sixty (60) milligrams of PMGC were dissolved into 15 ml of NaOH. The solution was placed into the oven. Afterwards, it was let to cool down at room temperature. The pH was neutralized using 1:2 (V/V) hydrochloric acid. The presence of free amino groups in the PMGC was estimated through the OPA technique, the result being the generation of 6.47 mM of amino groups.

### Example 7.2: N-Deacetylation of serogroup "A" Neisseria Meningitidis polysaccharide.

Thirty (30) milligrams of group "A" meningococcus polysaccharide (PMGA) were dissolved in 8 ml of NaOH. The solution was placed into the oven for the time established for this polysaccharide in the aforementioned example.. Afterwards, it was let to cool down at room temperature. The pH was neutralized with 1:2 (V/V) hydrochloric acid. The presence of free amino groups in the polysaccharide was estimated through the OPA technique, the result of which being the generation of 2.5 µmols of amino groups per ml of solution.

### Example 7.3: Coupling of the modified group "C" meningococcus polysaccharide with the activated group "A" meningococcus polysaccharide.

The solution consisting of 20 mg of PMGC and 3 ml of PBS with a pH of 7.4 which was modified as described in the Example 7.1 was added 30 mg of EDAC. It was stirred for 25 minutes at room temperature. After that we added the PMGA activated as described in the Example 7.2. The mixture was then stirred for 3 hours and after that time it was diafiltered against PBS at a pH of 7.4. Diafiltration was repeated 5 times against 50 ml of buffer every time. After the last diafiltration, it was left in 2 ml and stored at a temperature of 4° C until it was eventually used.

### Example 7.4: Coupling of conjugated polysaccharides with carrier proteins.

We added 50 mg of EDAC to 5 ml of a tetanus toxoid (TT) solution at a concentration of 1 mg / ml (estimated according to the Lowry method). It was stirred for 15 minutes at room temperature. Afterwards we added 2ml of the polysaccharide solution as obtained in the Example 7.3. Then it was stirred for 2 to 4 hours at room temperature. It was then dialyzed during the whole night at a temperature of 4° C against PBS with a pH of 7.4 (5L). We changed the buffer twice. The day after it was purified using a 16 x 100 mm column with Sepharose CL 4B. The buffer used to elute the peak was PBS at a pH of 7.4. We collected the first peak detected at 280 nm, at which we estimated the protein concentration through the Lowry method, using as a pattern the BSA. The PMGC content per sialic acid was estimated using the resorcinol method. The PMGA content was estimated by calculating the PO₄. Resulting conjugates were stored at a temperature of 4° C, with an additional 0.02 per cent of thimerosal up until they were eventually used.

**Table 9:**

| Results of the conjugation process. | | | | |
|---|---|---|---|---|
| Conjugate | PMGA (mg/ml) | PMGC (mg/ml) | TT (mg/ml) | PMGA / PMGC / TT Ratio |
| PMGA-PMGC - TT | 0.75 | 0.83 | 0.54 | 1.4/1.5/1 |
| PMGA = Group "A" meningococcus polysaccharide | | | | |
| PMGC = Group "C" meningococcus polysaccharide | | | | |

1. Conjugate (copiar la columna)
2. (copiar la columna)
3. )copiar la columna)
4. PMGA / PMGC / TT Ratio (copiar la columna)
PMGA = Group "A" meningococcus polysaccharide
PMGC = Group "C" meningococcus polysaccharide

### Example 7.5: Immunization procedure

Groups of 8 white male Balb/c mice (8-10 weeks old) were intraperitoneally inoculated with 2 dose (0, and 28 days; each dose contained a polysaccharide concentration of 10ug of PMGC) of the conjugate prepared as described in the Example 7.4. Before each inoculation and 14 days after the last inoculation, we extracted blood from the animals in order to obtain the sera that would be further submitted to immunogenicity tests.

### Example 7.6: Estimation of mice antibodies through the ELISA method.

This technique was applied with the use of Maxisorp plates (Nunc) with a poly-L lysin coating (100 :I / well) and was let to incubate for 1 hour at room temperature in a humid chamber. After that, the plate was washed four times with PBS, pH 7.4, plus 0.5 ml of Tween-20 dissolved in one liter of buffer solution. It was then coated with a solution of PMGC or PMGA (5 :g / ml) (depending on what the interest may be), with an addition of 100 :I / well, and it was let to incubate for 4 hours in a humid chamber at a temperature of 4° C. The plate was then washed four times with PBS (pH 7.4) and 0.5 ml of Tween-20 dissolved in one liter of buffer solution. It was then coated with a solution of glutaraldehyde (in PBS at 0.015 per cent), with an addition of 100 :I / well. Then it was let to incubate for 1 hour in a humid chamber at a temperature of 37° C. The plate was then washed four times with PBS (pH 7.4) plus 0.5 ml of Tween-20 dissolved in one liter of buffer solution and then it was coated with a gel solution (100 :g / ml) with an addition of 100 :I / well. It was let to incubate for 1 hour in a humid chamber at a temperature of 37° C. The plate was then washed four times and, as established by the protocol, we added 100 :I / well of serum (a dilution of 1 / 800) from each sample. It was left to incubate for 1 hour in a humid chamber at a temperature of 37° C. The plate was then washed four times. We added 100 :I / well of the second antibody, which consisted of one mice anti-IgG produced in ram, conjugated to peroxidase (Sigma No. 3742). It was let to rest for 1 hour at a temperature of 37° C. Later on, after washing the plate we added the substrate 1 (OPD + H₂O₂). It was let to rest for 15 minutes at room temperature, and the reaction was stopped by adding 50 :I / well of sulphuric acid 2N. The DO values were read at 492 nm on a Titertek Multieskam equipment.

### Brief description of Figures

Figure 1: Estimation of IgG anti- PMGC antibodies
   This Figure shows the way in which the absorbency values at 492 nm obtained from the serum of the immunized animals (3 dose after 0, 14, and 28 days) with the group "C" meningococcus polysaccharide (PMGC) activated with 0.7N of NaOH and diffused by means of a covalent bond with the tetanus toxoid and the VME are superior as compared to those obtained from the conjugate that contains P64K as a carrier protein. In general, absorbency values obtained for the three conjugates are superior to those obtained for the native polysaccharide. This is the first evidence of a change in the thymus-dependence of the PMGC after conjugation through the method described above.

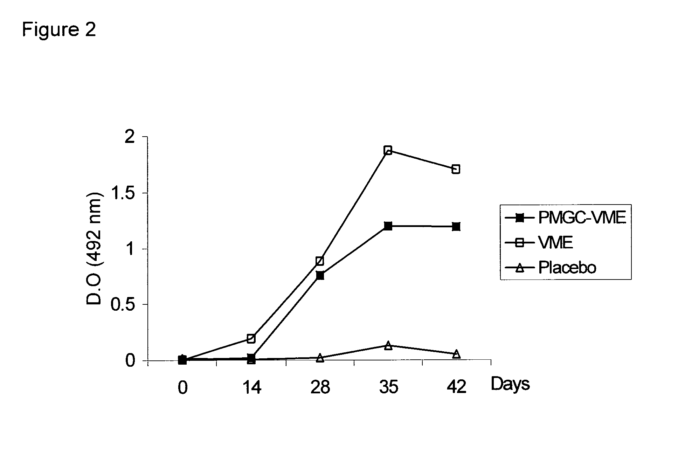

Figure 2: Estimation of IgG anti-VME antibodies.
   This Figure shows the results obtained when detecting the presence of IgG anti-VME antibodies in the serum of immunized animals with: Placebo, VME, and the conjugate obtained with the PMGC and these proteins. As can be seen, after the conjugation, titres of IgG anti-VME antibodies in the serum of immunized animals remained to be high. Although we can perceive a small reduction in the absorbency values as compared to the serum of animals immunized with the VME, we can affirm that with the conjugation procedure applied, the VME antigenic properties do not suffer any relevant damage.
Figure 3: Estimation of IgG anti-PMGC subclasses (IgG1, IgG2a)
   This Figure shows the results obtained when estimating the IgG anti-PMGC subclasses (IgG1, IgG2a) in the samples of the serum of mice which were administered the conjugates of PMGC with TT and VME obtained as a result of the application of our procedure. Likewise, it shows the results obtained in the serum animals immunized with the native PMGC. Here we can see, first, that there is a correlation between the results obtained through this technique and the results of the estimation of IgG antibodies. As can be seen, the titres obtained for the conjugates are much higher than those obtained in the case when the native PMGC was used. Besides, it can be seen that in the samples of mice immunized with the conjugates there is an increase in the titres of the IgG2a. These values are significantly higher in the conjugate where VME is the carrier protein. Based on these results we could affirm that there is a change not only in the thymus-dependence of the polysaccharide once it is conjugated; when the conjugation takes place with the VME, this induces a cellular T-helper (Th) 1 pattern in the presence of the polysaccharide, which is contrary to what the TT does, namely, to induce a Th2 pattern in the presence of the polysaccharide once it is conjugated.
Figure 4: Estimation of IgG anti-VME subclasses (IgG1, IgG2a)
   This Figure shows the results obtained in the estimation of IgG anti-VME subclasses (IgG1, IgG2a) in the serum of animals immunized with the VME and the PMGC-VME conjugate. Results shown in this Figure coincide with the results observed in Figure 2, which show the results of the estimation of IgG present in the serum of animals administered VME or the the PMGC-VME conjugate, since titres here are high. Likewise, the Figure shows the way in which VME has very high IgG2a values, with a Th1 response pattern. In the serum of animals immunized with the the PMGC-VME conjugate we can also see that despite reducing the absorbency values, there is still a Th1 response pattern for the VME.
Figure 5: Estimation of bactericidal antibodies against serogroup "C" N. Meningitidis.
   Results shown in this figure clearly show that the animal serum that contains the conjugate that is being tested is able to generate bactericidal antibody levels against serogroup "C" N. Meningitidis which are higher than the levels generated by the native PMGC, which indicates that the conjugate obtained is protectogenic.

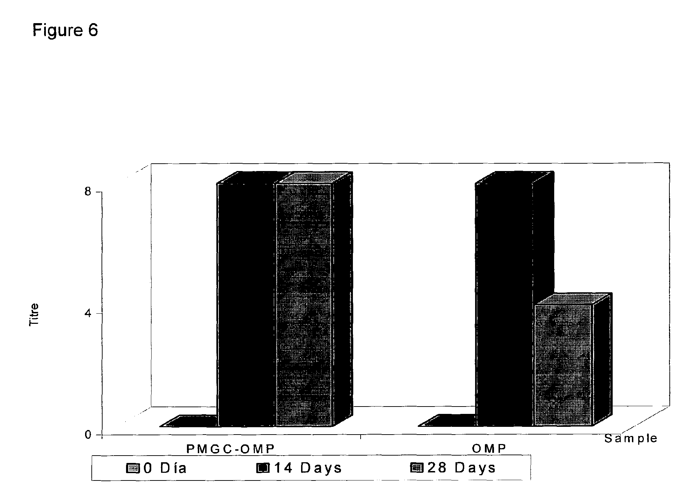

Figure 6: Estimation of bactericidal antibodies against serogroup "B" N. Meningitidis.
   This Figure shows the results of the estimation of bactericidal antibodies against serogroup "B" N. Meningitidis. As can be seen, 14 days after the animals were inoculated with the first dose, the conjugate show a bactericidal activity which is similar to the one obtained with the VME. We may see here how this bactericidal activity persists in the serum of the animals which were inoculated with the conjugated, as different from the case in which the VME was used. Here we may see a trend towards a decrease. This result shows that, as in the case of the serogroup "C" N. Meningitidis, this conjugate protects against serogroup "B" N. Meningitidis.
Figure 7: Estimation of the antigenicity of the LPS conjugated with TT.
   As is shown in this Figure, the antibodies present in the serum of human beings immunized with the attenuated strain of V. Cholerae recognized the conjugate obtained in our laboratory and showed absorbency values higher than those obtained for the samples of the native LPS (492 nm).
Figure 8: Estimation of IgG anti-V. Cholerae- LPS antibodies
   As is shown in this Figure, the serum of the animals immunized with the conjugate LPS-TT obtained in our laboratory showed absorbency values which were higher than those obtained from the serum of animals immunized with the LPS and the placebo.
Figure 9: Estimation of IgG anti-polysaccharide-Vi antibodies
   In the results shown by Figure 9, we saw that with a first dose we obtained an anti-Vi IgG response which was higher for the groups of animals immunized with the native Vi polysaccharide as compared to the groups immunized with the conjugates Antibodies titres for these last groups of animals increased after 28 days, when they were administered a second dose, until the maximum absorbency value was reached in time 35, while the group of animals immunized with the native Vi polysaccharide did not show any increase after the second dose. These results evidence the existence of a secondary immune response in the animals which were immunized with the conjugates. We may also see that in time 56 (28 days after the second dose) absorbency values are higher for the conjugate as compared to the native Vi polysaccharide, which suggests the idea that the response of IgG antibodies obtained with the conjugate is much longer than the one obtained with the non-conjugated Vi polysaccharide. These results corroborate what has been described in the literature, in the sense that with the conjugation process used in the creation of vaccines it is possible to change the TD of the polysaccharide used.
Figure 10: Estimation of IgG anti-PMGC antibodies (conjugates PRP-PMGC-TT).
   As is shown in this Figure, when we estimated the generation of IgG anti-serogroup "C" N. Meningitidis-polysaccharide antibodies in the serum of mice we realized that the absorbency levels obtained in the serum of animals immunized with the conjugates are higher than the absorbency values obtained for the rest of the samples that are being studied (serum of mice immunized with PBS and serum of mice immunized with the serogroup "C" N. Meningitidis native polysaccharide). We can also see that there is sero-conversion in the immune response induced by the conjugate,. Since the response obtained is higher than twice as much the original response.

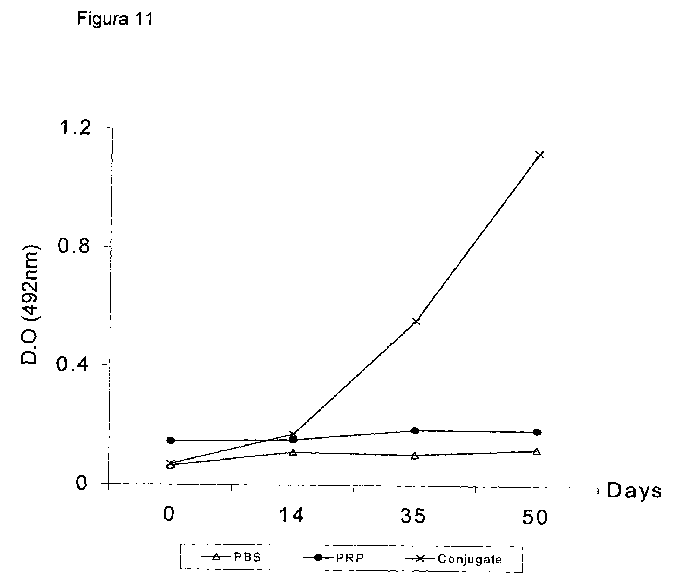

Figure 11: Estimation of IgG anti-PRP antibodies (conjugates PRP-PMGC-TT).
   As is shown in the Figure, when we estimated the generation of IgG anti-PRP antibodies in the serum of the mice that were being studied, the response found in the serum of the animals immunized with the conjugate were higher after the 14^{th} day, when they were inoculated the second dose. Here in this study we can also see that there is sero-conversion of the immune response against Type B H. Influenzae.

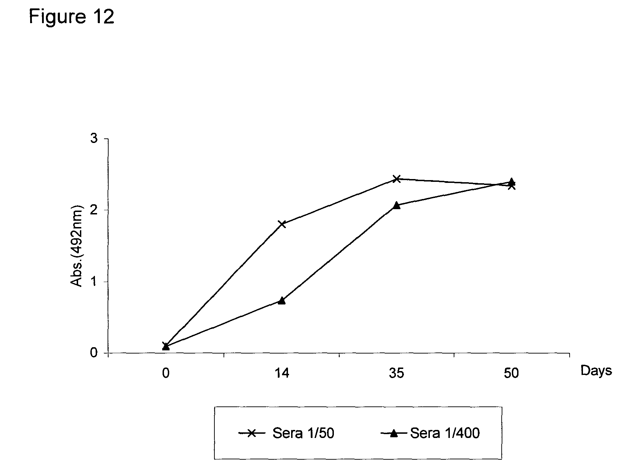

Figure 12: Estimation of IgG anti-TT antibodies (conjugates PRP-PMGC-TT).
   The Figure shows the estimation of IgG anti-TT antibodies generated in the serum of mice under study. Here we can see that the anti-TT response in animals immunized with the conjugate is high, which makes us think that the protein was not affected during the conjugation process and maintains its immunogenic properties.
Figure 13: Estimation of IgG anti-PMGC antibodies subclasses (conjugates PRP-PMGC-TT).
   As the Figure shows when we estimated the generation of IgG1 anti-PMGC antibodies in the serum of mice, we obtained very high absorbency values in the serum of the mice which were immunized with the conjugate, higher as compared to the absorbency values obtained for the samples of the serum of animals immunized with the native serogroup "C" N. Meningitidis polysaccharide. N the case of absorbency values obtained for the IgG 2^{a}, while we realize there is a slight increase in T3 (for Var I) this is very small, and the IgG1 type response prevails. Like in previous examples, we may see there is sero-conversion of the immune response induced by the conjugate for the IgG, since the response obtained is higher than twice as much the original response.
Figure 14: Estimation of IgG anti-PRP antibodies subclasses (conjugates PRP-PMGC-TT).
   In this Figure we may see that when we estimated the generation of IgG1 anti-type "B" H. Influenzae polysaccharide antibodies in the serum of mice, something similar to the aforementioned experience occurs, although for this polysaccharide the values obtained are slightly lower than those obtained when using the serogroup "C" N. Meningitidis polysaccharide, which was to be expected, since we already know that the serogroup "C" N. Meningitidis polysaccharide is in itself a better immunogenic as compared to the type "B" H. Influenzae polysaccharide. Nevertheless, as the Figure shows, the absorbency values obtained from the serum of mice immunized with the conjugate are much higher than those obtained from the serum of mice immunized with the PRP. In the case of the absorbency values obtained for the IgG 2a, while we can see that there is a slight increase of T3 (for Var I), such increase is very small. The IgG1 type response prevailed. As was the case in former examples, we can conclude that there is a seroconversion of the immune response induced by the conjugate for the IgG1, since the response obtained is higher than twice as much the original response.

### Advantages of the method proposed.

The pH and temperature levels used in this method reduce the possibilities of fragmentation of the polysaccharide, as well as the possible harm to the antigenic properties. With this invention we eliminate the formation of tetra butyl ammonium salts of polysaccharide when the conjugation takes place. Likewise, it prevents going through multiple stages to derivatize them, which makes this method to be applied in a short time an at a low cost.

The process of activation of antigens for a trivalent vaccine includes a few steps. It is not necessary to use complex molecules such as the 2-(6 aminocapropyl) 4,9-dioxo-1, 12-diaminododecane-naphtalene-1,5-disulphonic acid salt (ACA-DODAD-NDSA) and the S-acetyl mercapto succinic anhydride (SAMSA), or the intermediate purification steps necessary for the elimination of residual reagents. All in all, there is a trend towards an increased yield of antigens, as well as a decrease in the number of exogenous substances present in the vaccine formula, which are not of any interest. All of this favors the elimination of intermediate controls during the whole process and in the final vaccine product.

## Claims

1. Method to obtain multivalent vaccine conjugates, which includes the following steps:
a. Treatment of a first saccharide antigen in a basic medium, at a concentration of the base of 0.1 to 0.9 N, at a temperature between 40° C to 110° C for 3 to 10 hours;
b. Activate the carboxyl groups of that first antigen with carbodiimide;
c. Conjugate the activated carboxyl groups of the antigen obtained at step (b) with the second antigen that could be a protein or a saccharide, previously activated or not;
d. Purification of the conjugate obtained at step (c);
e. Activate of the carboxyl groups, using the carbodiimide, of a third antigen proteinaceous;
f. Conjugate the amino group presents in the saccharide antigens of the step (d) with the protein activated in step (e) or conjugate the amino group presents in the saccharide antigen of the step (a) with the protein activated in step (e);
g. Purification of the conjugate obtained in step (f).

2. Method, according to claim 1, **characterized by** the treatment of the first saccharide antigen, the base medium could be, selected out of the groups NaOH or KOH, or LiOH.

3. Method, according to claim 1, **characterized by** the first saccharide antigen contains amide-like bonds.

4. Method, according to claim 3, **characterized by** the first saccharide antigen comes from a bacterium.

5. Method, according to claim 4, **characterized by** the first saccharide antigen could be selected from a bacterium of the gender *Neisseria, Salmonella* or *Vibrio.*

6. Method, according to claim 5, **characterized by** the first saccharide antigen come from bacteria of the gender *Neisseria* is from *N. meningitidis* species.

7. Method, according to claim 6, **characterized by** the saccharide antigen could be selected from *N. meningitidis* serogroup C, *N. meningitidis* serogroup B or *N. meningitidis* serogroup A.

8. Method, according to claim 5, **characterized by** the saccharide antigen comes from bacteria of the gender *Salmonella* is from *Salmonella typhi* species.

9. Method, according to claim 8, **characterized by** the saccharide antigen is the Vi polysaccharide.

10. Method, according to claim 5, **characterized by** the saccharide antigen comes from bacteria of the gender *Vibrio* is from *Vibrio cholerae* species.

11. Method, according to claim 10, **characterized by** the saccharide antigen is the *V. cholera* lipopolysaccharide.

12. Method, according to claim 1, **characterized by** the second antigen is a proteinaceous type could be a protein or a peptide.

13. Method, according to claim 12, **characterized by** such antigen could be obtain through natural, recombinant, or synthetic procedures.

14. Method, according to claim 13, **characterized by** such antigen comes from virus, fungi, plants, animals or bacteria.

15. Method, according to claim 14, **characterized by** such antigen could be selected from bacteria of the gender *Escherichia, Salmonella, Shigella, Vibrio, Neisseria* or *Clostridium.*

16. Method, according to claim 15, **characterized by** such antigen comes from bacteria of the gender *Escherichia* is the *E. coli* species.

17. Method, according to claim 15, **characterized by** such antigen comes from bacteria of the gender *Salmonella* is the *S. typhi* species.

18. Method, according to claim 15, **characterized by** such antigen comes from a bacteria of the gender *Shigella* is the *Shigella sonnei.*

19. Method, according to claim 15, **characterized by** such antigen comes from bacteria of the gender *Vibrio* is the *V. cholera* species.

20. Method, according to claim 19, **characterized by** such antigen is the subunit B of the cholera toxin (CTB).

21. Method, according to claim 15, **characterized by** such antigen comes from a bacteria of the gender *Neisseria* is the *N. meningitidis* species.

22. Method, according to claim 21, **characterized by** such antigen comes from *N. meningitidis* serogroup B.

23. Method, according to claim 15, **characterized by** such antigen comes from bacteria of the gender *Clostridium* is the *C. tetanic* species.

24. Method, according to claim 23, **characterized by** the protein is the tetanic toxoid.

25. Method, according to claim 1, **characterized by** the second antigen is a saccharide, that comes from a bacterium.

26. Method, according to claim 25, **characterized by** the saccharide antigen could be selected from bacteria of the gender *Haemophilus, Neisseria* or *Vibrio.*

27. Method, according to claim 26, **characterized by** such saccharide antigen comes from bacteria of the gender *Haemophilus* is the *H. influenzae* species.

28. Method, according to claim 27, **characterized by** such saccharide antigen comes from *Haemophilus influenzae* type b.

29. Method, according to claim 28, **characterized by** the saccharide antigen is polyribosyl ribitol phosphate (PRP).

30. Method, according to claim 1, **characterized by** when the second saccharide antigen is the PRP, this must be activated with the 1,8 di-amino octane.

31. Method, according to claim 26, **characterized by** the saccharide antigen comes from bacteria of the gender *Neisseria* is the *N. meningitidis* species.

32. Method, according to claim 31, **characterized by** such saccharide antigen could be selected from *N. meningitidis* serogroup C, *N. meningitidis* serogroup B or *N. meningitidis* serogroup A.

33. Method, according to claim 32, **characterized by** the saccharide antigen could be the *N. meningitidis* serogroup C polysaccharide, *N. meningitidis* serogroup B lipopolysaccharide or *N. meningitidis* serogroup A polysaccharide.

34. Method, according to claim 26, **characterized by** the saccharide antigen comes from bacteria of the gender *Vibrio* is the *V. cholerae* species.

35. Method, according to claim 34, **characterized by** the saccharide antigen is the *V. cholerae* lipopolysaccharide.

36. Method, according to claim 1, **characterized by** the third antigen of proteinaceous type could be obtain by natural, recombinant, or synthetic procedures.

37. Method, according to claim 36, **characterized by** the third antigen of proteinaceous type comes from virus, fungi, plants, animals or bacteria.

38. Method, according to claim 37, **characterized by** such protein could be selected from bacteria of the gender *Salmonella, Neisseria, Clostridium or Shigella.*

39. Method, according to claim 38, **characterized by** such protein comes from bacteria of the gender *Salmonella* is the *S. typhi* species.

40. Method, according to claim 39, **characterized by** such antigens are proteins of the outer membrane.

41. Method, according to claim 38, **characterized by** the protein comes from bacteria of the gender *Neisseria* is the *N. meningitidis* species.

42. Method, according to claim 41, **characterized by** the protein comes from *N. meningitidis* serogroup B.

43. Method, according to claim 42, **characterized by** such antigens are proteins of the outer membrane.

44. Method, according to claim 42, **characterized by** the protein is p64k of *N. meningitidis* serogroup B, obtained through recombinant procedures.

45. Method, according to claim 38, **characterized by** the protein comes from bacteria of the gender *Clostridium* is the *C. tetanic* species.

46. Method, according to claim 45, **characterized by** the protein is the tetanus toxoid.

47. Method, according to claim 38, **characterized by** the protein comes bacteria of the gender *Shigella* is the *Shigella sonnei* species.

48. Method, according to claim 47, **characterized by** such antigens are proteins of the outer membrane.

49. Multivalent vaccine composition obtained by the described method through claims 1 to 48 **characterized by** include a conjugated saccharide dissolved in a safe vehicle pharmaceutically

50. Multivalent vaccine composition according to claim 49, **characterized by** include 10 to 25µg of conjugated saccharide.

51. Vaccine composition, according to claim 49, **characterized by** the conjugate includes the *N. meningitidis* serogroup C polysaccharide and this could be covalently united to tetanus toxoid, protein p64k of *N. meningitidis* serogroup B obtained by recombinant procedure or outer membrane proteins from *N. meningitidis* serogroup B.

52. Vaccine composition, according to claim 49, **characterized by** the conjugate includes the *V. cholerae* lipopolysaccharide and this could be covalently united to subunit B of the toxin of *V. cholerae* or tetanus toxoid.

53. Vaccine composition, according to claim 49, **characterized by** the conjugate includes the Vi polysaccharide and this could be covalently united to tetanus toxoid, proteins from *Shigella* or proteins from *Salmonella.*

54. Vaccine composition, according to claim 49, **characterized by** the conjugate could be compound by *N. meningitidis* serogroup A polysaccharide or by *Neisseria meningitidis* serogroup B lipopolisaccharide covalently united to tetanus toxoid.

55. Vaccine composition, according to claim 49, **characterized by** the conjugate includes *Neisseria meningitidis* serogroup C polysaccharide covalently united to the tetanus toxoid and the poly-ribosyl ribitol phosphate (PRP).

56. Vaccine composition, according to claim 49, **characterized by** the conjugate includes the Vi polysaccharide covalently united to proteins from *Shigella* and proteins from *Salmonella.*

57. Vaccine composition, according to claim 49, **characterized by** the conjugate includes the Vi polysaccharide covalently united to proteins from *Shigella* and lipopolysaccharide from *V. cholerae.*

58. Vaccine composition, according to claim 49, **characterized by** the conjugate includes *Neisseria meningitidis* serogroup C polysaccharide covalently united to tetanus toxoid and *Neisseria meningitidis* serogroup A polysaccharide.

59. The use of a Vaccine composition according to claims from 49 to 58 for the protection of illnesses caused by bacteria such as, *Neisseria meningitidis* serogroups A, B or C, *S. typhi, V. cholerae, Shigella* or tetanus toxoid.
